# EUROPEAN PATENT APPLICATION

(11) **EP 4 369 348 A1**
(43) Date of publication of application: **15.05.2024**
(21) Application number: 22206858.7
(22) Date of filing: 11.11.2022
(51) Int. Cl.: G16H 10/00, G16H 10/60

(54) **METHODS AND SYSTEMS FOR PREDICTING PERFORMANCE DRIFT OF CLINICAL PRESICTION MODELS**

(71) Applicant: Koninklijke Philips N.V., 5656 AG Eindhoven (NL)
(72) Inventor: OP DEN BUIJS, Jorn, Eindhoven (NL); MANS, Ronny Servatius, Eindhoven (NL)
(74) Representative: Philips Intellectual Property & Standards

(57) **Abstract**

A method (100) for monitoring a change in a predictive model of a system (270), comprising: receiving (120) patient data for input parameters for the predictive model; generating (130) a distribution of the received patient data; mapping (140) to each input parameter, one or more medical devices utilized to obtain the received patient data for the respective input parameter to generate an equipment map; updating (150) the equipment map to generate an updated equipment map after receiving an update to the medical devices; analyzing (160), by a self-learning model, the updated equipment map and the generated distribution to predict that a change in the medical devices will result in a predicted change of: (i) the distribution of the received patient data; (ii) an output of the predictive model; or (iii) an algorithm performance metric; determining (170) that the predicted change exceeds a predetermined range or threshold; and providing (180) an alert.

## Description

### FIELD OF THE INVENTION

The present disclosure is directed generally to methods and systems for predicting changes to the output of one or more predictive models of a clinical decision support (CDS) system.

### BACKGROUND OF THE INVENTION

Machine learning has been widely applied to build clinical prediction models for clinical decision support tools. Examples of clinical prediction models include models utilized to predict hospital length of stay, readmission, disease diagnosis, survival, and prognosis, clinical deterioration, exacerbation of a medical condition, and much more. The goal of these models is to timely provide a patient with the best treatment, intervention, or diagnosis, and/or to provide the patient with insight into their prognosis. In addition, clinical prediction models may aid the care provider in planning and maximizing their resources.

Since clinical prediction models drive important decisions in clinical care and affect the lives of millions of patients, it is extremely important that these models perform well. However, at least one study has suggested that there is an "AI chasm" between the promise of AI and the reality of AI. This chasm can be at least partly attributed to a lack of reliability monitoring, which can be defined as monitoring the performance of a clinical prediction model after operationalization. Ideally, warning signs about any issue with the performance of a clinical prediction should be provided before that issue arises. However, one challenge is that the methodology for measuring the input information for a clinical prediction model is often different across hospitals, and can even change over time in the same hospital. This can be related to both the equipment used, as well as the way of working with that equipment, among other possible changes to the system.

Further, the current state-of-the-art for monitoring the performance of clinical predictive models is by means of comparing their prediction outputs to the actual clinical outcomes. This comparison is captured by performance metrics such as the area under the receiver operator characteristic curve (AUC), sensitivity, and specificity. However, these metrics require many samples before a change is recognized. Thus, for example, if a change occurs in the means of measuring a heart rate (e.g., a different device is used with a higher degree of accuracy, or a different sampling rate), and this results in a change of the input features and eventually the clinical prediction model performance, it may take several days/weeks before sufficient samples are collected to indicate a change in AUC, sensitivity, or specificity.

### SUMMARY OF THE INVENTION

Accordingly, there is a continued need for methods and systems capable of predictively monitoring performance of a clinical prediction model. Various embodiments and implementations herein are directed to a clinical analysis system configured to monitor a change in a predictive model of a clinical decision support (CDS) system. The clinical analysis system receives patient data for an input parameter of the predictive model of the CDS service and generates a distribution of the received patient data for the input parameter. The clinical analysis system also generates an equipment map by mapping, to the input parameter, the medical device(s) utilized to obtain the patient data for that input parameter. The clinical analysis system updates the equipment map to generate an updated equipment map in response to receiving an update to the inventory of the plurality of medical devices. The system then analyzes, using a self-learning model, the updated equipment map and the generated distribution of the received patient data to predict that a change in the medical device(s) will result in a predicted change of one or more of: (i) the distribution of the received patient data for the one or more input parameters; and (ii) an output of the predictive model of the CDS service. If the system determines that the predicted change exceeds a predetermined range or threshold for change, the system generates and provides an alert via a user interface.

Generally, in one aspect, a method for monitoring a change in a predictive model of a clinical decision support (CDS) system is provided. The method includes: (i) receiving, by the clinical analysis system over a first time period, patient data for one or more input parameters for the predictive model of the CDS service; (ii) generating, by the clinical analysis system for the first time period, a distribution of the received patient data for the one or more input parameters; (iii) mapping, by the clinical analysis system, to each of the one or more input parameters, one or more medical devices utilized to obtain the received patient data for the respective input parameter to generate an equipment map, wherein the clinical analysis system comprises an inventory of a plurality of medical devices utilized to obtain patient data, and wherein the inventory of the plurality of medical devices is updated when any of the plurality of medical devices is changed or modified; (iv) updating, by the clinical analysis system, the equipment map to generate an updated equipment map in response to receiving an update to the inventory of the plurality of medical devices; (v) analyzing, by a self-learning model of the clinical analysis system, the updated equipment map and the generated distribution of the received patient data for the one or more input parameters to predict that a change in the one or more medical devices utilized to obtain the received patient data for the respective input parameter will result in a predicted change of one or more of: (1) the distribution of the received patient data for the one or more input parameters; (2) an output of the predictive model of the CDS service; and (3) an algorithm performance metric; (vi) determining, by the clinical analysis system, that the predicted change exceeds a predetermined range or threshold for change; and (vii) providing, via a user interface of the clinical analysis system, an alert that the predicted change exceeds the predetermined range or threshold for change.

According to an embodiment, the generated distribution of the received patient data for the one or more input parameters comprises a minimum, maximum, mean, standard deviation, and/or range of the patient data.

According to an embodiment, the received update to the inventory of the plurality of medical devices comprises an expected future change to a medical device.

According to an embodiment, the alert further comprises information about one or more of the predicted change and the predetermined range or threshold for change that is being exceeded. According to an embodiment, the alert further comprises information about the medical device that is updated in the inventory of the plurality of medical devices.

According to an embodiment, the patient data is received from an electronic health record database.

According to an embodiment, the change or modification of a medical device comprises one or more of a software or hardware update of the medical device, a change of a setting of the medical device, and replacement of the medical device.

According to an embodiment, the method further includes storing one or more of the generated distribution of received patient data for the one or more input parameters, the equipment map, and the updated equipment map.

According to another aspect is a system for monitoring a change in a predictive model of a clinical decision support (CDS) system. The system includes: patient data for one or more input parameters for the predictive model of the CDS service; a self-learning model; a processor configured to: (i) receive the patient data for one or more input parameters for the predictive model of the CDS service; (ii) generate a distribution of the received patient data for the one or more input parameters; (iii) map, to each of the one or more input parameters, one or more medical devices utilized to obtain the received patient data for the respective input parameter to generate an equipment map, wherein the clinical analysis system comprises an inventory of a plurality of medical devices utilized to obtain patient data, and wherein the inventory of the plurality of medical devices is updated when any of the plurality of medical devices is changed or modified; (iv) update the equipment map to generate an updated equipment map in response to receiving an update to the inventory of the plurality of medical devices; (v) analyze, using the self-learning model, the updated equipment map and the generated distribution of the received patient data for the one or more input parameters to predict that a change in the one or more medical devices utilized to obtain the received patient data for the respective input parameter will result in a predicted change of one or more of: (1) the distribution of the received patient data for the one or more input parameters; (2) an output of the predictive model of the CDS service; and (3) an algorithm performance metric; and (vi) determine that the predicted change exceeds a predetermined range or threshold for change; and a user interface configured to provide an alert that the predicted change exceeds the predetermined range or threshold for change.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
FIG. 1 is a flowchart of a method for monitoring a change in a predictive model of a clinical decision support (CDS) system, in accordance with an embodiment.
FIG. 2 is a schematic representation of a clinical analysis system, in accordance with an embodiment.
FIG. 3 is a flowchart of a self-learning model, in accordance with an embodiment.
FIG. 4 is a flowchart of a method for monitoring a change in a predictive model of a clinical decision support (CDS) system, in accordance with an embodiment.

### DETAILED DESCRIPTION OF EMBODIMENTS

The present disclosure describes various embodiments of a system and method configured to monitor performance of a clinical prediction model. More generally, Applicant has recognized and appreciated that it would be beneficial to provide a clinical analysis system configured to monitor a change in a predictive model of a clinical decision support (CDS) system. The clinical analysis system receives patient data for an input parameter of the predictive model of the CDS service and generates a distribution of the received patient data for the input parameter. The clinical analysis system also generates an equipment map by mapping, to the input parameter, the medical device(s) utilized to obtain the patient data for that input parameter. The clinical analysis system updates the equipment map to generate an updated equipment map in response to receiving an update to the inventory of the plurality of medical devices. The system then analyzes, using a self-learning model, the updated equipment map and the generated distribution of the received patient data to predict that a change in the medical device(s) will result in a predicted change of one or more of: (i) the distribution of the received patient data for the one or more input parameters; and (ii) an output of the predictive model of the CDS service. If the system determines that the predicted change exceeds a predetermined range or threshold for change, the system generates and provides an alert via a user interface.

The embodiments and implementations disclosed or otherwise envisioned herein can be utilized with any patient care system, including but not limited to clinical decision support tools, among other systems. For example, one application of the embodiments and implementations herein is to improve analysis systems such as, e.g., the Philips^{®} IntelliSpace^{®} and Patient Flow Capacity Suite products (manufactured by Koninklijke Philips, N.V.), among many other products. However, the disclosure is not limited to these devices or systems, and thus disclosure and embodiments disclosed herein can encompass any device or system capable of monitoring performance of a clinical prediction model.

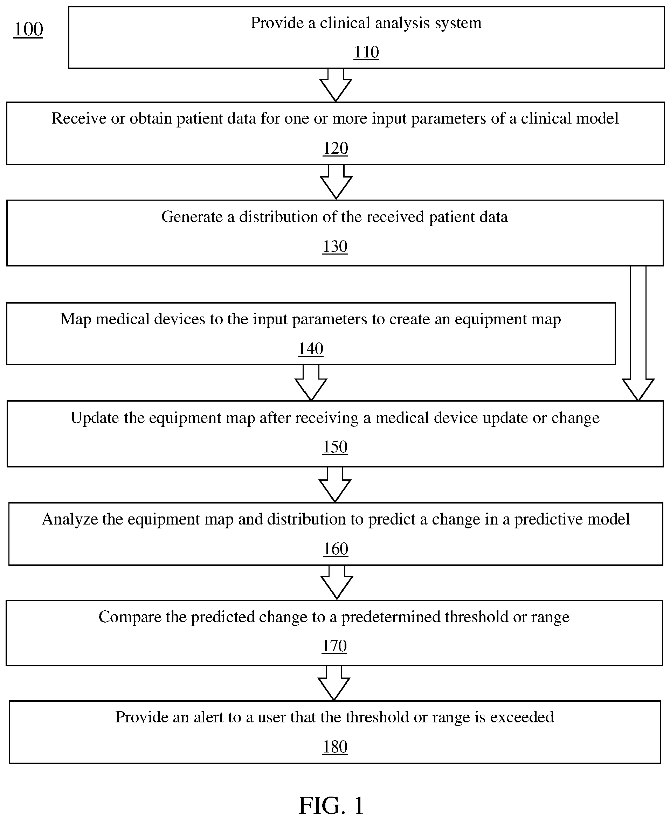

Referring to FIG. 1, in one embodiment, is a flowchart of a method 100 for monitoring a change in a predictive model of a clinical decision support (CDS) system, using a clinical analysis system. The methods described in connection with the figures are provided as examples only, and shall be understood not to limit the scope of the disclosure. The clinical analysis system can be any of the systems described or otherwise envisioned herein. The clinical analysis system can be a single system or multiple different systems.

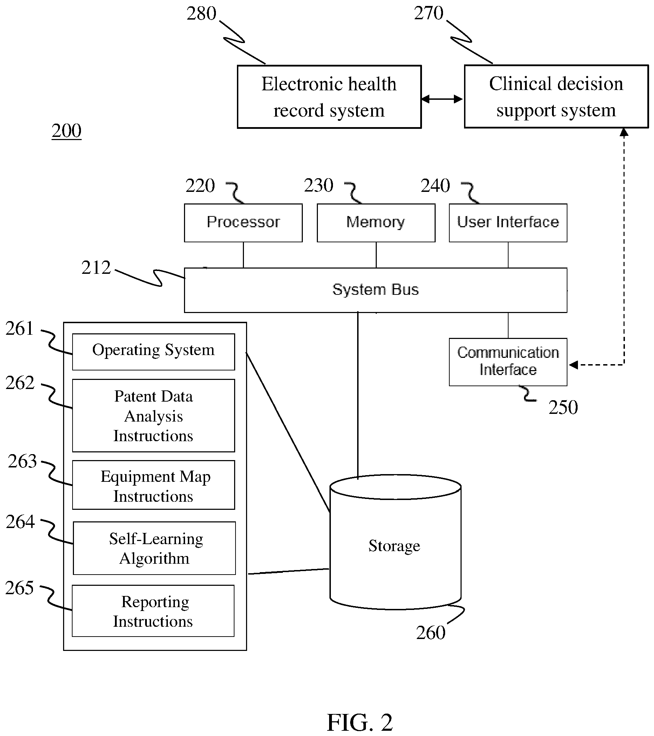

At step 110 of the method, a clinical analysis system is provided. Referring to an embodiment of a clinical analysis system 200 as depicted in FIG. 2, for example, the system comprises one or more of a processor 220, memory 230, user interface 240, communications interface 250, and storage 260, interconnected via one or more system buses 212. It will be understood that FIG. 2 constitutes, in some respects, an abstraction and that the actual organization of the components of the system 200 may be different and more complex than illustrated. Additionally, clinical analysis system 200 can be any of the systems described or otherwise envisioned herein. Other elements and components of the clinical analysis system 200 are disclosed and/or envisioned elsewhere herein.

According to an embodiment, the clinical analysis system comprises or is in communication with a local or remote Clinical Decision Support (CDS) system or service 270. The CDS system or service 270 can be any system or service that supports or otherwise facilitates decision-making by a clinician. For example, the CDS system or service can be configured to utilize patient data and/or other input to generate feedback, recommendations, summaries, and/or other information that can be utilized by the clinician for decision-making. According to another embodiment, the CDS system or service can support operational excellence including outside the hospital setting, supporting for example operational decision-making to expedite patients going throughout the hospital and where/when they should be discharged to after they leave the hospital, among many other decisions. Thus, the CDS system or service 270 need not be labeled a "CDS" system or service, but can instead be any system or service that supports or otherwise facilitates decision-making by a clinician, care facility, or other entity.

According to an embodiment, the CDS system or service comprises one or more clinical predictive models, classifiers, or algorithms. A clinical predictive model is trained to receive input data and generate a predictive output. There are a wide variety of clinical predictive models available and possible. As a few non-limiting examples of clinical predictive models, there are models configured to predict hospital length of stay, likelihood of readmission, disease diagnosis, disease survival, disease prognosis, treatment pathways, clinical deterioration, exacerbation of a medical condition, and much more.

According to an embodiment, the clinical analysis system and/or the CDS comprises or is in communication with an Electronic Health Record (EHR) system or service 280. The EHR system or service 280 can be, for example, a database of health information and records for one or more patients. The EHR database can be any database comprising health records for a multitude of patients or subjects. The EHR system or service 280 may be a local or remote system and can be in direct and/or indirect communication with the clinical analysis system and/or the CDS.

The health information and records stored in the EHR database can comprise any data about a patient. For example, the patient records may comprise demographic information, diagnostic information, clinical measurements, treatment information, and/or any other information, including over a period of time for each of the plurality of patients. According to an embodiment, the patient records comprises clinical data that measures patient physiology - such as physiological measurements of vital signs, lab results, imaging results, and other data - and event logs - such as timestamps of orders of labs, imaging, medication, consultation, and other data - that can reflect the procedures the patient received in a care setting. The patient records can comprise any other information. Thus, the EHR system or service can also be, for example, a recipient and/or source of past and current medical data for a patient, such current patient monitoring data, vital sign data, and much more.

According to an embodiment, the CDS system or service utilizes a trained clinical predictive model to analyze received input data and generate a predictive output. This can be triggered by a request from a clinician, a predetermined event, data received from the EHR, or via a variety of other triggering events. According to an embodiment, in response to a trigger the CDS system or service receives, fetches, or otherwise obtains the necessary input data and analyzes that input data with the trained clinical predictive model to generate the output. According to one embodiment, the output can be provided to a user, and/or can be returned to the EHR such as in the form of CDS cards which can be a small snippet of mark-up language, among other options.

At step 120 of the method, the clinical analysis system 200 receives, retrieves, or otherwise obtains patient data for one or more input parameters for a predictive model of the CDS service or system. A predictive model, such as a trained clinical prediction model, utilizes data for one or more input parameters in order to generate the programmed or trained output. The data can be patient data, equipment data, environmental data, user data, or any other data for any input parameter for which the predictive model is trained or programmed to utilize in order to generate the output.

Accordingly, the patient data for the one or more input parameters can be any data utilized by any predictive model. As just a few non-limiting examples, the input parameters can be measurements such as body temperature, blood glucose, blood gasses, blood pressure, heart rate, and a wide variety of other measurements and patient information inputs. Essentially any information about the patient and/or the patient's environment may be information that can be measured or otherwise qualified or quantified and utilized as an input parameter for one or more of a wide variety of predictive models.

As described in greater detail elsewhere herein, the patient data measured or obtained for the input parameters is measured or obtained by medical devices or equipment. As just a few non-limiting examples, body temperature may be measured by a thermometer, blood glucose may be measured by a glucose meter, blood gasses may be measured by a blood gas analyzer, blood pressure may be measured by a blood pressure monitor, heart rate may be measured by a heart rate monitor, and many, many more. Notably, these medical devices or equipment measure or obtain the patient data in a particular way, including with regard to frequency and other variable parameters. Thus, changes in these variable parameters may change the patient data received for the one or more input parameters, and thus impact the prediction model that utilizes these input parameters.

According to an embodiment, the patient data can be received, retrieved, or otherwise obtained by the clinical analysis system 200 from any source. The source may be the CDS system or service, and/or an EHR system or database that is in communication with the CDS system or the clinical analysis system. The patient data can be continually or periodically fed into or requested by the clinical analysis system. The patient data may be temporarily stored in a local or remote database before being utilized by the clinical analysis system.

According to an embodiment, the patient data is received, retrieved, or otherwise obtained by the clinical analysis system for the duration of a first time period. The first time period can be any time period sufficient to allow downstream analysis of the received patient data. For example, depending on the nature of the patient data, the time period can be very short (such as seconds or minutes or hours) or the time period can be very long (such as days or weeks or months). Data that is sampled more frequently may require a shorter time period, and data that is sampled less frequently may require a longer time period.

At step 130 of the method, the clinical analysis system generates a distribution of the received patient data for the one or more input parameters of a predictive model for a first time period. The distribution can be, for example, the minimum, maximum, mean, standard deviation, and/or range of the patient data for an input parameter over the course of the first time period, although other analyses are possible. The first time period can be a single time period, or a distribution can be generated cumulatively and/or in windows.

According to an embodiment, once the distribution of the received patient data for the one or more input parameters of a predictive model is generated, the distribution can be utilized immediately, or it can be stored in local and/or remote database for subsequent downstream use by the system.

At step 140 of the method, the clinical analysis system generates an equipment map. The system maps one or more medical devices utilized to obtain received patient data for a respective input parameter, to that respective input parameter. Thus, the clinical analysis system comprises an equipment map that identifies the medical device(s) utilized to generate, receive, or otherwise obtain the patient data for a respective input parameter. The equipment map may comprise any information about a medical device, including model, manufacturer, settings, and/or any other information about the device. As described in greater detail herein, these medical devices or equipment measure or obtain the patient data in a particular way, including with regard to frequency and other variable parameters. Changes in these variable parameters may change the patient data received for the one or more input parameters, and thus impact the prediction model that utilizes these input parameters. Accordingly, knowing which medical device(s) can affect or impact which input parameters, and potentially how the medical device(s) affect or impact an input parameter, is beneficial information for the clinical analysis system.

The generated equipment map can be utilized immediately, or it can be stored in local and/or remote database for subsequent downstream use by the system.

According to an embodiment, the clinical analysis system comprises or is in communication with an inventory of a plurality of medical devices utilized to obtain patient data. For example, the inventory may be a database of medical devices within a care setting such as a hospital, outpatient facility, long-term care facility, and/or other care environment. The inventory may comprise an identification of the medical devices within that setting, along with information about the devices including the model, manufacturer, settings, location, and/or any other information about the device. The inventory may be stored in a local or remote memory of the clinical analysis system, or may be a database with which the clinical analysis system is in communication.

According to an embodiment, the database is or is a component of an inventory management system that tracks details of hospital medical equipment including details such as type, model, manufacturer, location, settings, and so on. The CDS system can access this information, such as via an Application Programming Interface (API). This information can be utilized by the CDS service to track - such as tracking changes over time - medical equipment parameters that are related to the input parameters of a clinical prediction model. In the event of a change to a piece of equipment, the CDS service can alert a user to indicate that this might affect the performance of the clinical prediction model involved. Furthermore, in the event of change, the user is also alerted that the mapping of medical equipment to input data needs updating, or the mapping is automatically adjusted. The change can be any change including but not limited to a replacement of the device, a software or hardware update of the device, a change in a setting of a device, and/or any other change.

According to an embodiment, the inventory or database of medical devices is updated when any of the inventoried device is changed. The update may be automated, such as when an inventoried device is configured or otherwise designed to push information about an update or representative of an update to the inventory or database. The update may be manual, such as when a user manually updates information about an inventoried device in the inventory or database of medical devices when that inventoried device is changed. Again, the change can be any change including but not limited to a replacement of the device, a software or hardware update of the device, a change in a setting of a device, and/or any other change.

At step 150 of the method, the clinical analysis system updates the equipment map to generate an updated equipment map. According to an embodiment, the updated equipment map is generated in response to receiving an update to the inventory or database of medical devices. According to an embodiment, generating an updated equipment map can be simply updating information about one device in the map, such as updating a setting for the device, the location of the device, the make or model of the device, and/or any other information. Generating the updated equipment map may comprise updating information about a plurality of devices, including adding devices, removing devices, changing which devices are mapped to which input parameters, and a variety of other changes.

According to an embodiment, the clinical analysis system is configured to generate an updated equipment map only if the update to the inventory or database of medical devices affects an inventoried device that is mapped to an input parameter of a predictive model of the CDS system or service. In other words, if the piece of equipment in the inventory (i.e., an inventoried device) that is changed is also utilized to collect patient data for an input parameter, the clinical analysis system may then be triggered to generate an updated equipment map. Alternatively, if the inventoried device that is changed is not utilized to collect patient data for an input parameter, the clinical analysis system may not be triggered to generate an updated equipment map.

The update to the inventory or database of medical devices can be automatically detected by the clinical analysis system. For example, the clinical analysis system may periodically or continually monitor the inventory in order to determine when there are changes to the inventory. Alternatively, the clinical analysis system may receive an indication from a user, from the CDS, or from the inventory or database that there has been a change to the inventory.

Once generated, the updated equipment map can be utilized immediately, or it can be stored in local and/or remote database for subsequent downstream use by the system.

At step 160 of the method, the clinical analysis system analyzes the updated equipment map and the generated distribution of the received patient data for the one or more input parameters. The clinical analysis system analyzes the information using a self-learning model. The self-learning model can be any model, algorithm, or program configured or trained to utilize the provided output - such as the updated equipment map and the generated distribution(s) - to generate an output. According to an embodiment, the output of the self-learning model is a prediction that a change to one of more of the medical devices in the equipment map, as reflected in the updated equipment map, will result in a predicted change in the distribution of patient data, a change in the output of a predictive model of the CDS service, and/or a change in the predictive model performance metrics (such as AUC, sensitivity, and/or specificity) especially if clinical outcomes are available. The change to one of more of the medical devices in the equipment map, as reflected in the updated equipment map, can be any detectable change, including but not limited to adding a device, removing a device, changing which device is mapped to an input parameters, modification of device settings, aging of a device, and a wide variety of other changes.

It may be appreciated that alerting on any changes in equipment parameters related to the input parameters may result in large numbers of false alerts. Therefore, according to an embodiment, the self-learning model can use a prediction model itself to predict parameters related to the distributions of the clinical prediction model inputs, outputs, and/or performance. For example, the self-learning model can be used to predict whether a change in equipment will also cause a change in: (i) a distribution of one or more of the input parameters; (ii) a distribution of a prediction score of the predictive algorithm; and/or (iii) a predictive algorithm performance metric such as area under the receiver operator characteristic curve (AUC), sensitivity, and specificity. If any of these are predicted to have a variation above, below, or otherwise outside a predetermined threshold, then the user may be alerted. According to an embodiment, the self-learning model is continuously updated such as through underlying machine learning technology that can deal with time-series, such as auto-regressive moving average models, and recurrent neural networks. The model can be used to bootstrap prediction and alerting of possible clinical prediction model drift in different hospitals.

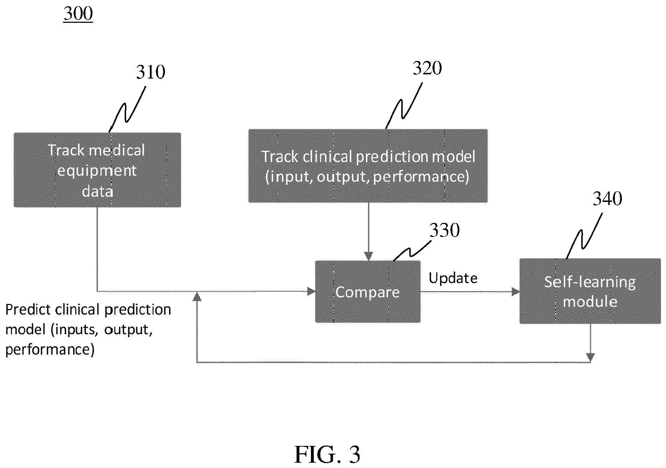

Referring to FIG. 3, in one embodiment, is a flowchart 300 of the self-learning model of the clinical analysis system. At 310, the clinical analysis system tracks medical equipment data with the equipment map, which can be updated and modified. The equipment map can be utilized to for the clinical prediction model for analysis of inputs, output, and performance. At 320, the clinical analysis system tracks information about the clinical prediction model, such as a generated distribution of received patient data for one or more input parameters for the clinical prediction model. At step 330, the system can compare the equipment map and/or tracked information about the clinical prediction model to each other or to previous versions of themselves in order to determine if there has been any change. At 340, the self-learning model can update and learn and improve, such as through underlying machine learning technology that can deal with time-series, such as auto-regressive moving average models, and recurrent neural networks.

At step 170 of the method, the clinical analysis system determines that the predicted change in the distribution of patient data, the predicted change in the output of a predictive model of the CDS service, and/or the predicted change in an algorithm performance metric exceeds a predetermined range or threshold for change. For example, the system may be programmed with or may learn to comprise a threshold for change. For example, a threshold may be 5% or greater for a predicted change in the distribution of the one or more input parameters, or 10% or more, or an actual amount rather than a percentage, or any other threshold or range. Alternatively, the system may utilize feedback from a user to learn when a predicted change should exceed a threshold or range. For example, if an alert is provided but that alert is dismissed by a user, then the system may learn that this predicted change was not sufficiently different to result in an alarm. Once the clinical analysis system determines that the predicted change in the distribution of patient data and/or the predicted change in the output of a predictive model of the CDS service exceeds a predetermined range or threshold for change, the system can trigger an alert to be provided to a user.

At step 180 of the method, the clinical analysis system provides, via a user interface, an alert that the predicted change in the distribution of patient data, a change in the output of a predictive model of the CDS service, and/or the predicted change in an algorithm performance metric exceeds the predetermined range or threshold for change. The alert can include information about the equipment map including but not limited to a change in a medical device that resulted in the alert, information about the one or more parameters and/or output of the predictive model that is predicted to change, and/or any other information. Reporting via the user interface can be done using any method for reporting. For example, the information may be communicated by wired and/or wireless communication to another device. For example, the system may communicate the information to a mobile phone, computer, laptop, wearable device, and/or any other device configured to allow display and/or other communication of the information. The reported information may be textual information, visual information, audible information, haptic information, or other method for communicating information.

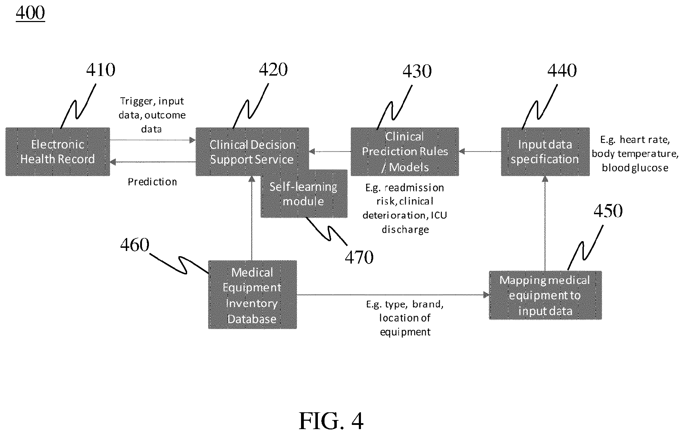

Referring to FIG. 4, in one embodiment, is a flowchart 400 of a method for monitoring, by a clinical analysis system 200, a change in a predictive model of a clinical decision support (CDS) system. The clinical analysis system comprises or is in communication with an electronic health record system or database 410 which comprises patient data. The patient data may trigger an analysis by the system, is utilized by the predictive algorithm of the CDS to generate predictions, and can be used for many other purposes. The clinical analysis system comprises or is in communication with the CDS 420, which comprises at least one clinical prediction algorithm 430. The clinical prediction algorithm can be utilized for any of a wide variety of clinical analyses, including but not limited to the analyses described or otherwise envisioned herein.

According to an embodiment, the clinical analysis system comprises an analysis 440 of patient data utilized as an input parameter of the clinical prediction algorithm 430 of the CDS. The analysis 440 can be, for example, a distribution of the received patient data for the one or more input parameters of a predictive model for a first time period. The distribution can be, for example, the minimum, maximum, mean, standard deviation, and/or range of the patient data for an input parameter over the course of the first time period, although other analyses are possible. The first time period can be a single time period, or a distribution can be generated cumulatively and/or in windows.

According to an embodiment, the clinical analysis system comprises an equipment map 450 which is generated from a medical equipment inventory database 460. The equipment map is a map of one or more medical devices utilized to obtain received patient data for a respective input parameter, to that respective input parameter. The equipment map may comprise any information about a medical device, including model, manufacturer, settings, and/or any other information about the device. As described in greater detail herein, these medical devices or equipment measure or obtain the patient data in a particular way, including with regard to frequency and other variable parameters. Changes in these variable parameters may change the patient data received for the one or more input parameters, and thus impact the prediction model that utilizes these input parameters. Accordingly, knowing which medical device(s) can affect or impact which input parameters, and potentially how the medical device(s) affect or impact an input parameter, is beneficial information for the clinical analysis system.

According to an embodiment, the self-learning model or module 470 of the clinical analysis system is configured to analyze the analysis 440 of patient data and the equipment map 450 to predict a change in output of the clinical prediction algorithm 430, preferably before the change in the output is detected or detectable. For example, a change to a device in the equipment map may affect the patient data obtained by that device, which may then change the input and/or output of the clinical prediction algorithm.

Referring again to FIG. 2, in one embodiment, is a schematic representation of a clinical analysis system 200. System 200 may be any of the systems described or otherwise envisioned herein, and may comprise any of the components described or otherwise envisioned herein. It will be understood that FIG. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.
It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, system 200 comprises or is in communication with a local or remote Clinical Decision Support (CDS) system or service 270. The CDS system or service 270 is any system or service that supports or otherwise facilitates decision-making by a clinician. For example, the CDS system or service is configured to utilize patient data and/or other input to generate feedback, recommendations, summaries, and/or other information that can be utilized by the clinician for decision-making. According to an embodiment, the CDS system or service comprises one or more clinical predictive models, classifiers, or algorithms. A clinical predictive model is trained to receive input data and generate a predictive output. There are a wide variety of clinical predictive models available and possible.

According to an embodiment, system 200 and/or CDS 270 comprises or is in communication with a local or remote EHR system 280, which is an electronic medical records database from which the information about a plurality of patients, including demographic, diagnosis, and/or treatment information may be obtained or received. According to an embodiment, the EHR system 280 is an EHR database from which patient data is obtained. The EHR database may be a local or remote database and is in direct and/or indirect communication with system 200. Thus, according to an embodiment, clinical analysis system 200 and/or the CDS 270 system comprises an EHR database or system 280.

According to an embodiment, storage 260 of system 200 may store one or more algorithms, modules, and/or instructions to carry out one or more functions or steps of the methods described or otherwise envisioned herein. For example, the system may comprise, among other instructions or data, patient data analysis instructions 262, equipment map instructions 263, a self-learning model or algorithm 264, and/or reporting instructions 265.

According to an embodiment, patient data analysis instructions 262 direct the system to generate an analysis of received patient data for one or more input parameters of a predictive model of the CDS for a first time period. The clinical analysis system 200 receives, retrieves, or otherwise obtains patient data for one or more input parameters for the predictive model of the CDS service or system. The data can be patient data, equipment data, environmental data, user data, or any other data for any input parameter for which the predictive model is trained or programed to utilize in order to generate the output. As described in greater detail elsewhere herein, the patient data measured or obtained for the input parameters is measured or obtained by medical devices or equipment. The clinical analysis system generates a distribution of the received patient data for the one or more input parameters of a predictive model for a first time period. The distribution can be, for example, the minimum, maximum, mean, standard deviation, and/or range of the patient data for an input parameter over the course of the first time period, although other analyses are possible. The first time period can be a single time period, or a distribution can be generated cumulatively and/or in windows. According to an embodiment, once the distribution of the received patient data for the one or more input parameters of a predictive model is generated, the distribution can be utilized immediately, or it can be stored in local and/or remote database for subsequent downstream use by the system.

According to an embodiment, equipment map instructions 263 direct the system to generate an equipment map. The system maps one or more medical devices utilized to obtain received patient data for a respective input parameter, to that respective input parameter. Thus, the clinical analysis system comprises an equipment map that identifies the medical device(s) utilized to generate, receive, or otherwise obtain the patient data for a respective input parameter. The equipment map may comprise any information about a medical device, including model, manufacturer, settings, and/or any other information about the device. As described in greater detail herein, these medical devices or equipment measure or obtain the patient data in a particular way, including with regard to frequency and other variable parameters. The generated equipment map can be utilized immediately, or it can be stored in local and/or remote database for subsequent downstream use by the system.

According to an embodiment, self-learning model or algorithm 264 analyzes the updated equipment map and the generated distribution of the received patient data for the one or more input parameters. The self-learning model can be any model, algorithm, or program configured or trained to utilize the provided output - such as the updated equipment map and the generated distribution(s) - to generate an output. According to an embodiment, the output of the self-learning model is a prediction that a change to one of more of the medical devices in the equipment map, as reflected in the updated equipment map, will result in a predicted change in the distribution of patient data, a change in the output of a predictive model of the CDS service, and/or the predicted change in an algorithm performance metric. The change to one of more of the medical devices in the equipment map, as reflected in the updated equipment map, can be any detectable change, including but not limited to adding a device, removing a device, changing which device is mapped to an input parameters, modification of device settings, aging of a device, and a wide variety of other changes.

According to an embodiment, reporting instructions 265 direct the system to generate and provide a user via a user interface information comprising an alert that a predicted change in the output of a clinical prediction algorithm is subject to change as a result of a change to a medical device or other change in the system. The information may be communicated by wired and/or wireless communication to another device. For example, the system may communicate the information to a mobile phone, computer, laptop, wearable device, and/or any other device configured to allow display and/or other communication of the information.

According to an embodiment, the clinical analysis system is configured to process many thousands or millions of datapoints in the generation of the input parameter distribution, in the generation of the equipment map, and in the analysis of the input parameter distribution and equipment map by the self-learning model. For example, generating the input parameter distribution and the equipment map, and then analyzing those things with the self-learning model, requires processing of millions of datapoints from input data and the resulting analysis. This can require millions or billions of calculations to generate the input parameter distribution and the equipment map, as well as a novel self-learning model, from those millions of datapoints and millions or billions of calculations. As a result, each input parameter distribution and equipment map, as well as the self-learning model, is novel and distinct based on the input data and parameters of the clinical analysis system and the corresponding CDS. Thus, generating a functional and skilled trained self-learning algorithm, which relies on the generated input parameter distribution and the equipment map, comprises a process with a volume of calculation and analysis that a human brain cannot accomplish in a lifetime, or multiple lifetimes.

In addition, the self-learning model is configured to continually receive new inputs, perform the analysis, and provide periodic or continual updates via the information provided to a user for the patient. This requires the analysis of thousands or millions of datapoints on a continual basis to optimize the reporting, requiring a volume of calculation and analysis that a human brain cannot accomplish in a lifetime.

By providing this novel clinical analysis system as described or otherwise envisioned herein, the improved CDS system or service using this clinical analysis system has an enormous positive effect on patient analysis and care compared to prior art systems.
All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for monitoring, by a clinical analysis system (200), a change in a predictive model of a clinical decision support (CDS) system (270), comprising:
receiving (120), by the clinical analysis system over a first time period, patient data for one or more input parameters for the predictive model of the CDS service;
generating (130), by the clinical analysis system for the first time period, a distribution of the received patient data for the one or more input parameters;
mapping (140), by the clinical analysis system, to each of the one or more input parameters, one or more medical devices utilized to obtain the received patient data for the respective input parameter to generate an equipment map, wherein the clinical analysis system comprises an inventory of a plurality of medical devices utilized to obtain patient data, and wherein the inventory of the plurality of medical devices is updated when any of the plurality of medical devices is changed or modified;
updating (150), by the clinical analysis system, the equipment map to generate an updated equipment map in response to receiving an update to the inventory of the plurality of medical devices;
analyzing (160), by a self-learning model of the clinical analysis system, the updated equipment map and the generated distribution of the received patient data for the one or more input parameters to predict that a change in the one or more medical devices utilized to obtain the received patient data for the respective input parameter will result in a predicted change of one or more of: (i) the distribution of the received patient data for the one or more input parameters; (ii) an output of the predictive model of the CDS service; and (iii) an algorithm performance metric;
determining (170), by the clinical analysis system, that the predicted change exceeds a predetermined range or threshold for change; and
providing (180), via a user interface of the clinical analysis system, an alert that the predicted change exceeds the predetermined range or threshold for change.

2. The method of claim 1, wherein the generated distribution of the received patient data for the one or more input parameters comprises a minimum, maximum, mean, standard deviation, and/or range of the patient data.

3. The method of claim 1, wherein the received update to the inventory of the plurality of medical devices comprises an expected future change to a medical device.

4. The method of claim 1, wherein the alert further comprises information about one or more of the predicted change and the predetermined range or threshold for change that is being exceeded.

5. The method of claim 1, wherein the alert further comprises information about the medical device that is updated in the inventory of the plurality of medical devices.

6. The method of claim 1, wherein the patient data is received from an electronic health record database (280).

7. The method of claim 1, wherein the change or modification of a medical device comprises one or more of a software or hardware update of the medical device, a change of a setting of the medical device, and replacement of the medical device.

8. The method of claim 1, further comprising the step of storing one or more of the generated distribution of received patient data for the one or more input parameters, the equipment map, and the updated equipment map.

9. A system (200) for monitoring a change in a predictive model of a clinical decision support (CDS) system (270), comprising:
patient data for one or more input parameters for the predictive model of the CDS service;
a self-learning model (264);
a processor (220) configured to: (i) receive the patient data for one or more input parameters for the predictive model of the CDS service; (ii) generate a distribution of the received patient data for the one or more input parameters; (iii) map, to each of the one or more input parameters, one or more medical devices utilized to obtain the received patient data for the respective input parameter to generate an equipment map, wherein the clinical analysis system comprises an inventory of a plurality of medical devices utilized to obtain patient data, and wherein the inventory of the plurality of medical devices is updated when any of the plurality of medical devices is changed or modified; (iv) update the equipment map to generate an updated equipment map in response to receiving an update to the inventory of the plurality of medical devices; (v) analyze, using the self-learning model, the updated equipment map and the generated distribution of the received patient data for the one or more input parameters to predict that a change in the one or more medical devices utilized to obtain the received patient data for the respective input parameter will result in a predicted change of one or more of: (1) the distribution of the received patient data for the one or more input parameters; and (2) an output of the predictive model of the CDS service; and (3) an algorithm performance metric; and (vi) determine that the predicted change exceeds a predetermined range or threshold for change; and
a user interface (240) configured to provide an alert that the predicted change exceeds the predetermined range or threshold for change.

10. The system of claim 9, wherein the generated distribution of the received patient data for the one or more input parameters comprises a minimum, maximum, mean, standard deviation, and/or range of the patient data.

11. The system of claim 9, wherein the received update to the inventory of the plurality of medical devices comprises an expected future change to a medical device.

12. The system of claim 9, wherein the alert further comprises information about one or more of the predicted change and the predetermined range or threshold for change that is being exceeded.

13. The system of claim 9, wherein the alert further comprises information about the medical device that is updated in the inventory of the plurality of medical devices.

14. The system of claim 9, wherein the patient data is received from an electronic health record database 280.

15. The system of claim 9, wherein the change or modification of a medical device comprises one or more of a software or hardware update of the medical device, a change of a setting of the medical device, and replacement of the medical device.
